# EUROPEAN PATENT SPECIFICATION

(11) **EP 1 357 922 B1**
(45) Date of publication and mention of the grant of the patent: **25.05.2011**
(21) Application number: 02709917.5
(22) Date of filing: 07.02.2002
(51) Int. Cl.: A61K 35/36

(54) **CELL SUSPENSION PREPARATION TECHNIQUE AND USE**
ZELLSUSPENSIONSHERSTELLUNGSTECHNIK UND VERWENDUNG
TECHNIQUE DE PREPARATION DE SUSPENSION CELLULAIRE ET UTILISATION

(30) Priority: 07.02.2001 AU PR298901; 04.04.2001 US 281527 P
(43) Date of publication of application: 05.11.2003
(60) Divisional application: 10184235.9
(73) Proprietor: McComb Foundation Inc., Bentley, Western Australia 6102 (AU)
(72) Inventor: STONER, Marie, Bentley, Western Australia 6102 (AU); WOOD, Fiona, Bentley, Western Australia 6102 (AU)
(74) Representative: Dehmel, Albrecht
(86) International application number: PCT/AU2002/000120
(87) International publication number: WO 2002/062358

(56) References cited:
- EP-A- 0 350 887
- EP-A- 0 444 270
- AU-A- 3 990 197
- US-A- 1 356 794
- US-A- 3 647 632
- US-A- 4 649 115
- US-A- 5 786 207
- US-A- 6 080 581
- HIROBE T: "Melanocyte stimulating hormone induces the differentiation of mouse epidermal melanocytes in serum-free culture." JOURNAL OF CELLULAR PHYSIOLOGY. UNITED STATES AUG 1992, vol. 152, no. 2, August 1992 (1992-08), pages 337-345, XP009025292 ISSN: 0021-9541
- OSBORNE C S ET AL: "Investigation into the biological stability of collagen/chondroitin-6-sulphate gels and their contraction by fibroblasts and keratinocytes: the effect of crosslinking agents and diamines." BIOMATERIALS. ENGLAND FEB 1999, vol. 20, no. 3, February 1999 (1999-02), pages 283-290, XP004168904 ISSN: 0142-9612
- PETERSEN M J ET AL: "Enhanced synthesis of collagenase by human keratinocytes cultured on type I or type IV collagen." THE JOURNAL OF INVESTIGATIVE DERMATOLOGY. UNITED STATES MAR 1990, vol. 94, no. 3, March 1990 (1990-03), pages 341-346, XP009025276 ISSN: 0022-202X
- NOËL-HUDSON M S ET AL: "Human epidermis reconstructed on synthetic membrane: influence of experimental conditions on terminal differentiation." IN VITRO CELLULAR & DEVELOPMENTAL BIOLOGY. ANIMAL. UNITED STATES 1995 JUL-AUG, vol. 31, no. 7, July 1995 (1995-07), pages 508-515, XP009025285 ISSN: 1071-2690
- NICKOLOFF B J ET AL: "Further characterization of the keratinocyte somatomedin-C/insulin-like growth factor I (SM-C/IGF-I) receptor and the biological responsiveness of cultured keratinocytes to SM-C/IGF-I." DERMATOLOGICA. SWITZERLAND 1988, vol. 177, no. 5, 1988, pages 265-273, XP009025282 ISSN: 0011-9075
- GUEDON I ET AL: "Culture and cytogenetic studies of adult human keratinocytes using a new growth factor." DIFFERENTIATION;RESEARCH IN BIOLOGICAL DIVERSITY. ENGLAND 1981, vol. 19, no. 2, 1981, pages 109-114, XP009025277 ISSN: 0301-4681
- ANONYMOUS: "Clinical Cell Culture- ReCell" INTERNET ARTICLE, [Online] 17 December 2003 (2003-12-17), XP002333821 Retrieved from the Internet: <URL:http://web.archive.org/web/2003121706 5921/http://www.clinicalcellculture.com/on ...> [retrieved on 2005-06-28]
- NAVARRO F.A. ET AL: 'Sprayed keratinocyte suspensions accelerate epidermal coverage in a porcine microwound model' J. BURN CARE REHABIL. vol. 21, no. 6, November 2000 - December 2000, pages 513 - 518, XP010307457
- GAUTHIER Y. ET AL: 'Autologous grafting with noncultured melanocytes: a simplified method for treatment of depigmented lesions' JOURNAL OF THE AMERICAN ACADEMY OF DERMATOLOGY vol. 26, no. PART 1, February 1992, ST. LOUIS, US, pages 191 - 194, XP000994526
- SOVENSJO T. ET AL.: 'Autologous keratinocyte suspension accelerate epidermal wound healing in pigs' J. SURG. RES. vol. 99, no. 2, August 2001, pages 211 - 221, XP002969741
- 'Product Number: 352360, 100 mum cell strainer' BD (BECTON, DICKINSON AND COMPANY) ONLINE CATALOGUE, [Online] pages 1 - 2, XP002969739 Retrieved from the Internet: <URL:http://catalog.bd.com/scripts/OBDsheet .exe?FNC=productlist_alistproducts_html_352 360> [retrieved on 2002-03-05]
- 'Product Number 352070, 50 ml centrifuge tubes' BD (BECTON, DICKINSON AND COMPANY) ONLINE CATALOGUE, [Online] pages 1 - 2, XP002969740 Retrieved from the Internet: <URL:http://catalog.bd.com/scripts/OBDsheet .exe?FNC=productlist_alistproducts_html_352 070> [retrieved on 2002-03-05]
- 'Product: dry block heat baths' G. KISKER-PRODUCTS FOR BIOTECHNOLOGY ONLINE CATALOGUE, [Online] pages 1 - 4, XP002969742 Retrieved from the Internet: <URL:http://www.kisker-biotech.com/block.ht m> [retrieved on 2002-03-05]

## Description

### FIELD OF THE INVENTION

This invention relates to a simple, rapid and cost effective technique for grafting of cells, and in particular to a method for preparing a suspension of cells from a tissue sample obtained from a donor site and applying that suspension of cells to a recipient site.

### BACKGROUND ART

There are many methods of treating wounds known to those skilled in the art. For example, skin grafting techniques exist which aim to reconstruct the skin covering areas of the body where there is either damage or defects to the skin. In general, these types of grafts are classified according to their host-donor relationship and by their thickness. The most clinically applied graft is the autologous graft, whereby tissue is taken from one area of the body and applied to another area. The grafted tissue then develops a new blood supply and attaches to the underlying tissues.

There are several types of skin grafts presently used, including split-thickness, full-thickness grafts, and micro-grafting. Each of these graft types must be prepared using certain techniques, and each one has its inherent advantages and disadvantages. Split-thickness grafts often require considerable skill, time and expensive equipment to perform. Further, donor sites are painful, result in scarring and limit the area able to be covered. Although they may be more successful than full-thickness grafts, they are usually less cosmetically attractive. Full-thickness grafts require less skill or expensive equipment, and their cosmetic appearance is better than that of split-thickness grafts. However, full-thickness grafts do not "take" as well as split-thickness grafts. Micro-grafts are more easily accomplished and require no special instruments. However, their cosmetic appearance is not as good as other techniques, as the resulting scarring is unacceptable.

A variation to the above grafting techniques is the mesh graft, which is a type of split-thickness or full-thickness skin graft in which parallel rows of slits are cut in the tissue being treated. Some of the advantages of mesh grafts include: a greater coverage of the effected area, drainage of blood or serum from under the graft, and increased conformity of the graft to uneven recipient areas. This technique has been very successful, with 90 to 100 per cent "take" when the grafts have been applied on healthy granulation beds.

An alternative to split-skin grafting is to form a blister under suction at a donor site and transplant to the recipient site. The production of blisters to treat wounds has been used since the 1960s. The blisters are produced by a suction device, such as Dermavac™, at a suction pressure of approximately 250-300 mmHg for 1-2 hours. The blisters are then cut off and placed on the wound. The healing time is around 10-14 days. There are several disadvantages of this method such as the amount of time required to prepare the graft is too long and the graft may not result in re-pigmentation of the area; or uneven pigmentation is common around the edges of the area of treatment.

Micro-grafting has become a more common approach for large area cover and involves the "snipping off" of a number of very small sections of tissue from a donor site and applying then to a dressing, which is in turn applied to the wound area.

The most advanced technology for the generation of a tissue *in vitro* is to culture epidermis. Cultured epithelial autografts (CEA) are an important adjunct in the coverage of burns and other situations in which large areas of the body's surface experience skin loss. There are many centres throughout the world with tissue culture facilities whose aim is to produce autologous epithelial grafts for use in a wide variety of applications. The usefulness and application of CEA is related to its ability to achieve confluent cells sheets suitable for grafting. This technique overcomes many of the disadvantages of the previous treatments described above. For example, cultured epithelial autografts reduce the demand for donor sites. However, these autografts are slow growing and require time for culturing of the grafts, which often exceeds the time of preparation of the recipient's sites.

The present invention provides a cellular suspension together with a method for preparing that suspension and a device for its preparation each of which seek to ameliorate one or more of the disadvantages associated with prior art grafting technology.

### SUMMARY OF THE INVENTION

The subject invention relates to a method for preparation of a unique cell suspension, which method is rapid, efficient and simple to prepare and apply. It also relates to the unique cell suspension for use in the treatment of patients in need of skin graft surgery.

According to a first aspect of the invention there is provided a method for preparing a cell suspension suitable for application to a patient, which method is as defined in attached claim 1.

In a preferred form of the invention the dissociating means is of a chemical nature such as an enzyme which is capable of disrupting cellular bonding like for example trypsin. Further, preferably the filtered cellular suspension is diluted to an appropriate cell density using a nutrient solution, which may be anything from a basic salt solution to a more complex nutrient solution.

According to a second aspect of the invention there is provided a cell suspension produced according to the above method for use in the treatment of patients as further specified in attached claim 7. Preferably the cells in the suspension are autologous cells (i.e. they are isolated from the patient requiring an autograft). The inventors have observed that by removing xenogenic serum from the cell suspension there is less likelihood of transmission of infection and xenogenic reactions between a patient and the serum are eliminated. Another feature of the cell suspension produced according to the above method is that the tissue sample used to isolate the cells in the suspension is removed from the enzyme solution before the cells are harvested. When cells are exposed to enzymes capable of damaging inter-cellular adhesion the viability of the cellular suspension decreases over time, thereby reducing the efficiency of the grafting when applied to a patient. The cell suspension produced according to the above method has been observed to possess greater cellular viability compared to comparative methods that harvest the cells at regular intervals whilst the tissue is immersed in the presence of enzymes like trypsin.

According to a third aspect of the invention there is provided a cellular suspension for use in the treatment of patients as further specified in attached claim 9.

### BRIEF DESCRIPTION OF DRAWINGS

**Figure 1** illustrates a perspective view of the apparatus used to prepare the cell suspension according to the present invention with the lid open and the second member in place.
**Figure 2** illustrates a perspective view of the apparatus used to prepare the cell suspension according to the present invention with the lid open and the second member removed and inverted.
**Figure 3a** illustrates a perspective view of the first member of the apparatus used to prepare the cell suspension according to the present invention
**Figure 3b** illustrates a perspective rear view of the first member of the apparatus used to prepare the cell suspension according to the present invention .
**Figure 4** illustrates a perspective view of the base of the apparatus used to prepare the cell suspension according to the present invention .

### DETAILED DISCLOSURE OF THE INVENTION

Throughout this specification and the claims that follow, unless the context requires otherwise, the word "comprise", or variations such as "comprises" or "comprising", will be understood to imply the inclusion of a stated integer or group of integers but not the exclusion of any other integer or group of integers.

### Description of Preferred Embodiments

Having regard to the above, this invention provides a method for producing a transplantable cellular suspension of living tissue suitable for grafting to a patient, as specified in attached claim 1. In applying the method, cells suitable for grafting back to a patient are collected and dispersed in a solution that is suitable for immediate dispersion over the recipient graft site.

The subject invention has many advantages over the prior art, some of which are illustrated in the following paragraphs.
1. It provides a time-efficient method for supplying a cellular cover to a skin tissue in a clinical setting. That is, cells are available when needed at the time of surgery. This is achievable because there is a very short preparation period of the cells, thus allowing grafting to be performed peri-operatively or in the rooms of a specialist physician or General Practitioner.
2. It provides a method which significantly reduces the complexity associated with the use of conventional CEA's and is particularly useful in cases of burn injury that have presented late. In some instances, cells are unavailable at the time of surgery, either due to delayed referral of a patient with an unhealed burn or simply because the time needed for culturing of the grafts had exceeded that for preparation of the recipient wound bed. The present invention ameliorates the issue of graft preparation time.
3. It aids in the achievement of rapid cell coverage in areas of injury and donor sites. It provides a means for reducing the size of donor sites - the biopsy donor site is markedly smaller than a split skin graft donor site and reduces or eliminates the use of split skin graft donor sites; improves expansion rate of cell coverage; improves the rate of healing of small burns; is useful for small areas of skin reconstructions, such as scars; and improves scar quality.
4. It ameliorates problems associated with the use of solutions used during conventional tissue culture process. According to the method of preparation, the cells used in a graft are suspended in a nutrient solution free of xenogenic serum. That suspension is then placed directly onto the recipient site.
5. It provides a means for the treatment of various skin disorders or diseases. For example, it may be used for the following: epidermal resurfacing, replacement after skin loss, site match-up during re-pigmentation of an area of skin, treatment of burn wounds, leukoderma, vitiligo, piebaldism, in the treatment of scars - for example, caused through incorrect wound healing, improper scar direction or scar distortion from wound contraction, acne scars; resurfacing cosmetic dermabrasion, resurfacing after laser treatment and in association with dermal reconstruction. Additionally the method may be used for cell replacement therapy, including epithelial cell (such as urothelial cell, buccal mucosal cell and respiratory epithelial cell) replacement treatment, and endothelial cell replacement treatment.
6. It provides a means to produce a suspension of cells in a ratio to each other comparable with those seen in situ. That is, due to the manner of preparation of the cellular suspension, cells such as keratinocyte basal cells, Langerhans cells, fibroblasts and melanocytes typically have enhanced survival rates in comparison to standard tissue culture techniques, whereby selective cell culture can result in the loss of certain cell types. This has the advantage of allowing for the correct re-pigmentation of skin after a skin graft.
7. It allows faster surgery and healing - thereby reducing trauma for patients during the phase of their medical care.

According to the first aspect of the invention there is provided a method for preparing a cell suspension suitable for use in resurfacing and regeneration of damaged skin tissue.

According to this method, tissue (preferably of an autologous nature) is harvested from a patient by means known in the art of tissue grafting. Preferably this is achieved by taking a tissue biopsy. With the harvesting of the biopsy consideration must be given to the depth of the biopsy and the surface area size. The depth and size of the biopsy influence the ease at which the procedure can be undertaken and speed with which a patient recovers from the procedure. In a highly preferred form of the invention the donor site should be chosen to appropriately match the recipient site, for example post-auricular for head and neck, thigh for lower limbs, inner-upper-arm for upper limbs, or palm for sole or vice-versa.

Once a biopsy has been harvested from a patient the tissue sample is subjected to physical and or chemical dissociating means capable of dissociating cellular stratum in the tissue sample.

Methods for dissociating cellular layers within the tissues are well known in the field. For example, the dissociating means may be either a physical or a chemical disruption means. Physical dissociation means might include, for example, scraping the tissue sample with a scalpel, mincing the tissue, physically cutting the layers apart, or perfusing the tissue. Chemical dissociation means might include, for example, digestion with enzymes such as trypsin, dispase, collagenase, trypsin-EDTA, thermolysin, pronase, hyaluronidase, elastase, papain and pancreatin. Non-enzymatic solutions for the dissociation of tissue can also be used.

Preferably, dissociation of the tissue sample is achieved by placing the sample in a pre-warmed enzyme solution containing an amount of enzyme sufficient to dissociate cellular stratum in the tissue sample. This may be achieved by, for example, using a trypsin solution, however, any other enzyme such as dispase, collagenase, trypsin-edta, thermolysin, pronase, hyaluronidase, pancreatin, elastase and papain that cause cells to become detached from other cells or from solid surfaces may be used for this purpose. When the enzyme used is trypsin the enzyme solution used in the method is preferably calcium- and magnesium-free. One such solution is preferably calcium- and magnesium ion-free phosphate buffered saline.

For a tissue biopsy derived from a patient's skin (comprising epithelial-dermal cells) the amount of trypsin that might be used in the method is preferably between about 5 and 0.1 % trypsin per volume of solution. Desirable trypsin concentrations of the solution are about 2.5 to 0.25%, with about 0.5% trypsin being most preferred.

The time period over which the tissue sample is subjected to the trypsin solution may vary depending on the size of the biopsy sample taken. Preferably, the tissue sample is placed in the presence of the trypsin solution for sufficient time to weaken the cohesive bonding between the tissue stratum. A tissue sample taken from a patient's skin might be placed in trypsin for a 5 to 60 minute period. Preferably, the tissue sample is immersed in the trypsin solution for between 10 and 30 minutes with 15 to 20 minutes being optimal for most tissue samples.

After the tissue sample has been immersed in the trypsin solution for an appropriate amount of time, the sample is removed from the trypsin and washed with nutrient solution. Washing the tissue sample may involve either partial or complete immersion of the treated sample in the nutrient solution. Alternatively, and more preferably, the wash solution is dripped on the tissue sample in sufficient volume to remove and or significantly dilute any excess trypsin solution from the surface of the sample. Preferably any dilution that might occur would lead to less than 0.05% trypsin in the nutrient solution.

The nutrient solution used in the method should be capable of significantly reducing and more preferably removing the effect of the trypsin either by dilution or neutralisation. The nutrient solution used in the method will also preferably have the characteristics of being (i) free of at least xenogenic serum, (ii) capable of maintaining the viability of the cells until applied to a patient, and (iii) suitable for direct application to a region on a patient undergoing tissue grafting. The solution may be anything from a basic salt solution to a more complex nutrient solution. Preferably, the nutrient solution is free of all serum but contains various salts that resemble the substances found in body fluids; this type of solution is often called physiological saline. Phosphate or other non-toxic substances may also buffer the solution in order to maintain the pH at approximately physiological levels. A suitable nutrient solution that is particularly preferred is Hartmann's solution.

After application of the nutrient solution to the tissue sample, the cellular stratum of the sample are separated permitting cells capable of reproduction to be removed from the cellular material and suspended in the nutrient solution. It is preferable that the dermis and epidermis are separated to allow access to the dermal-epithelial junction of both surfaces.

Cells capable of reproduction are then removed from the separated stratum by any means known in the art. Preferably, the reproductive cells are scraped off the surface of the stratum using an instrument such as a scalpel. Cells capable of reproduction within the dermal-epithelial junction include but are not limited to keratinocyte basal cells, Langerhans cells, fibroblasts and melanocytes. Following release of the cells from the tissue sample, they are suspended in the nutrient solution. Preferably only a small volume of nutrient solution is applied to the tissue sample during this harvesting step otherwise the suspension may become too fluid therein providing difficulties in applying the suspension to the graft.

To avoid excessively large cellular congregates in the cellular suspension the suspension is preferably filtered. Any filter capable of separating excessively large cellular congregates from the suspension may be used in this preferred step of the invention. In a highly preferred form of the invention the filter size is between 50µm and 200µm. More preferably, it is between 75µm and 150µm, with 100µm being one specific example.

Prior to application to the graft site or immediately after filtering, the cellular suspension may be diluted to produce an appropriate cell density suitable for the purpose to which the suspension is to be used.

According to the second aspect of the invention there is provided an aqueous cell suspension, produced by the method described in the first aspect of the invention, for use in the treatment of patients requiring skin graft surgery. The cell suspension provided by this method is highly suitable for tissue regeneration and grafting techniques. An important advantage of utilising such a suspension in grafting technology is that it can be used to greatly expand the area or volume of a wound that can be treated quickly by *in situ* multiplication of a limited number of cells. The number and concentration of cells seeded onto graft site may be varied by modifying the concentration of cells in suspension, or by modifying the quantity of suspension that is distributed onto a given area or volume of the graft site.

By suspending cells in a nutrient solution which is at least (i) free of xenogenic serum, (ii) capable of maintaining the viability of the cells until applied to a patient and (iii) suitable for direct application to a region on a patient undergoing tissue grafting, the inventors have found that the outcome of patient skin grafts is improved. A partial explanation for this appears to be attributable to the removal of xenogenic serum and more preferably all serum from the cell suspension. Xenogenic serum is a common additive in grafting culture medium and is well known to cause potential infective and hypersensitivity problems. Such serum is however generally required for the *in vitro* expansion of the cells and to neutralise the action of the enzyme if the enzyme used is trypsin. The nutrient solution used in the present invention does not require such serum because the cell population within the suspension is not expanded prior to application to the graft site. Rather cellular multiplication is encouraged on the patient rather than in an *in vitro* system. When trypsin is used, neutralisation is achieved by other means.

Another unique feature of the cell suspension produced according to the method of the first aspect of the invention is that the composition of cells in the cellular preparation is comparable to that seen in situ compared to prior art cellular preparation. One possible explanation for this is that in the prior art, culture of the cellular preparation utilises selective culture for keratinocytes, therefore loss of cellular constituents such as fibroblasts and melanocytes occurs, whereas the cellular suspension produced from the first aspect of the invention has a cell composition comparable to the in situ cell population. Another feature of the cellular suspension produced from the first aspect of the invention is that the graft cells are more viable as they are harvested in a nutrient solution as distinct from prior art cell harvesting procedures which utilise techniques where the cells are harvested whilst exposed to powerful digestive enzymes for excessive periods of time. When the cells are exposed to such enzymes for excessive periods of time, the viability of the cellular suspension decreases.

The invention allows for the treatment of patients requiring a skin tissue graft. By this method the cellular suspension produced according to the first aspect of the invention is applied to a graft site. A liquid suspension containing cells may be manually distributed onto the graft site by any of several techniques, which include spraying, spreading, pipetting and painting.

In a highly preferred form of the invention the suspension is to be sprayed on to a graft site. The suspension may be sprayed through any type of nozzle that transforms liquid into small airborne droplets. This embodiment is subject to two constraints. First, it must not subject the cells in solution to shearing forces or pressures that would damage or kill substantial numbers of cells. Second, it should not require that the cellular suspension be mixed with a propellant fluid that is toxic or detrimental to cells or wound beds. A variety of nozzles that are commonly available satisfy both constraints. Such nozzles may be connected in any conventional way to a reservoir that contains the cellular suspension.

Alternatively the suspension may be delivered via a pipette, common "eye-droppers," syringe and needle and/or other similar devices to place small quantities of cellular suspension on a graft site.

After the cell suspension has been applied to the recipient graft site, the wound may be dressed with a wound dressing. In a preferred embodiment the dressing is Surfasoft™, a woven nylon dressing. Preferably, the healing of the wound is followed up by standard protocols for skin graft treatment known to those skilled in the art.

An apparatus for developing a tissue regeneration solution has a heating means suitable for heating an enzyme solution to a required temperature and for maintaining that solution at the desired temperature for a suitable amount of time; and a filter recess comprising a filter means capable of separating large cellular congregates from a cellular suspension.

In a preferred form, the apparatus also includes a reservoir capable of holding a tissue sample and a nutrient solution which solution is also capable of maintaining the viability of the cells in the tissue sample. More preferably, the reservoir is of sufficient size to permit manipulation of the tissue sample permitting separation of the tissue cellular stratum and harvesting of those cells from the stratum suitable for grafting.

The apparatus may also include one or more fluid containment wells for storage of fluids such as the nutrient solution. The wells may alternatively serve as a receptacle for a container such as a plastic or glass vial that holds the nutrient solution. Preferably, the well is capable of holding at least a 10 ml volume. Such wells permit ease of fluid application to the tissue sample. Storage of such fluids in close proximity to its site of application also provides the advantage of reducing the risk of accidental leakage of the fluid and provides an easy means for accessing the fluid for accurately delivering it to either the tissue sample or the cell suspension.

In a highly preferred form, the apparatus comprises a first and second member wherein:
(1) the first member includes:
   (a) at least a heating means suitable for heating an enzyme solution to a required temperature and which is capable of maintaining that solution at the desired temperature for a suitable amount of time;
   (b) at least a filter recess comprising a filter means capable of separating large cellular congregates from a cellular suspension;
   (c) at least a fluid containment well for storage of nutrient solution;
(2) the second member forms a reservoir capable of withholding a tissue sample and nutrient solution in fluid containment; and
wherein the first member provides a seat upon which the second member may be placed during manipulation of the tissue.

In a further preferred form, the first member provides a storage compartment into which tools and solutions used in the above described method may be stored. Where such a compartment is provided in the apparatus, the second member may provide the lid or closure to that compartment. In use the lid is preferably removed from the top of the compartment and inverted. The underside of the lid preferably forms the reservoir therein enabling the second member to serve a dual purpose. Tools and solutions used in the method can be accessed from the compartment. The inverted lid may then be seated back over the compartment therein providing the reservoir for the apparatus.

The apparatus may be made from metal, plastic or any other material. Further, the container may be any size. Preferably, the size of the container is only limited by its intended use and the need for sterilisation such as by the use of gamma irradiation and or ethylene oxide.

It should be appreciated that the heating means employed in the apparatus may simply constitute a heating pad or pads on the top of the first member. There are however, attendant problems with such arrangements, not least of which is the possibility of accidental spillage of the container undergoing heating. Therefore, in an embodiment, one or more heating means may be housed within a recess in the first member. Also located within that recess is at least a container into which tissue may be placed for exposure to the enzyme solution. In an alternate embodiment, one or more heating means may be housed in the base of the apparatus. In such a configuration the first member contains at least an opening suitable for receiving a container capable of holding fluid, which opening provides access for the container to the heating means.

It will be appreciated that if the apparatus is designed for more than one use, the heating means may be capable of being repeatedly heated and cooled. Alternatively, each heating unit may be capable of a single use, but multiple heating units may be provided with the apparatus to facilitate multiple heating events.

In a highly preferred configuration of the apparatus, a heating collar is located within a recess therein forming a heating recess in the first member within which there is located a container (eg a vial) for the enzyme. The container is preferably held in place by at least a restraining means, which desirably surrounds part of the upper portion of the container preventing accidental release of the container from the apparatus. In circumstances, where the apparatus is intended for single use, the restraining means may be formed as an integral part of the first member, thus meaning that removal of the container may only be achieved by physically breaking the first member.

The heating means used in the apparatus is preferably controlled by circuitry permitting activation of the heating element when required. For example, the heating means may be switched on by depressing the start button located, for example, on the surface of the first member. Alternately, the heating means may be activated by pushing the container down with sufficient force to activate a switch located in the base of the apparatus. A person of ordinary skill in the field will appreciate that a wide range of electronic means may be used to activate the heating unit provided in the apparatus.

Desirably, the heating unit is also operably linked to a timer mechanism, which is adapted to heat the enzyme solution for a pre-defined period of time. In circumstances where the apparatus is intended for multiple uses, preferably the timer can be set to deactivate the heating element when a particular amount of time is reached. At which point an alarm may activate to inform the user that the time is up. The alarm may be audible or in the form of a light display.

In a further preferred configuration, the heating means may be provided with an adjustable temperature control. Where temperature adjustment is required, such variation may be achieved by adapting the heating control circuitry to include or communicate with a temperature control mechanism permitting the temperature of the heating unit to be constantly varied within a constant range, or it may present a range of selected temperatures that the heating control means can be set at. A temperature control means will beneficially be included in the apparatus where the apparatus is to be used in the harvesting and preparation of different cell types and/or where different enzymes are used in the harvesting method for which the apparatus has unique application.

In an alternate more preferred form, the apparatus is designed for single use. In such instances the timer mechanism is part of the circuitry that controls the heating means. Once the heating means has been activated it heats the solution for a pre-defined period of time and then self-destructs. It should be appreciated by those skilled in the art that such an the apparatus may be fitted with various monitoring means that are capable of indicating such things as: the enzyme has reached the required temperature; the amount of time that the enzyme has been in the solution for; and/or the amount of time left before the circuitry self-destructs etc. By way of example only, the monitoring means might consist of a series of LED's that activate when certain events occur. In a highly preferred embodiment, the heating element preferably remains in the heating mode for a maximum of 45 minutes to 1 hour.

The heating means may be powered by any means known in the art. Preferably, the power supply is provided by battery/batteries. In one form, the power supply is a battery or a plurality of batteries located in the base of the apparatus.

In a further form, the apparatus may be provided with one or more means to facilitate mixing of the solutions used in the invention, such as an enzyme solution. In this respect, and by way of example only, the apparatus may include a means for vortexing the solution; such as an electromagnetic system that is adapted to agitate a magnetic bead. Where the apparatus includes an electromagnetic mixing system, the magnetic bead is preferably provided in the container (eg vial) in which the solution is stored in the apparatus. Alternatively the magnetic bead may be added to the solution when mixing is desired.

In a highly preferred form, the mixing means is combined with the heating means either as a single unit or as separate units to facilitate constant heating of the solution in an even manner. Using such a mixing means avoids the possible overheating of solution closest to the heating unit while the solution is heated. Such a system will provide a more constant heating of the solution. Alternate means for mixing the solution will be known in the art and include, mechanical, physical, electrical and electromagnetic means as an example. While any mixing means may be employed in the apparatus, preferably the mixing means is either selected to minimise vibration of the apparatus or incorporated into the apparatus in a manner that minimise such vibration. In this respect the mixing means may be housed on one or more vibration dampeners or the apparatus may include one or more vibration dampeners on is base.

Where a mixing means is incorporated into the apparatus, the means may be automatically activated upon activation of the heating unit or, alternatively, there may be a separate activation system. Further, the speed of the mixing may either be fixed or variable. Preferably, there is a separate activation system for the mixing means.

The filter recess incorporated into the apparatus may be of any size or shape that facilitates filtering of a cellular suspension. Further, the recess may be adapted to receive and hold at least a tube into which cell the suspension may be filtered. Preferably, the recess has a conical base providing easy means to access the full volume of cell suspension after it has been filtered.

Desirably, the third recess is designed to receive a 100µm cell filter. The third recess can accommodate a 100µm cell filter connected to a conical tube. Preferably, the tube has area/volume graduations marked on the side.

The apparatus may also include a set of tools required for cell harvesting. It will be appreciated by those skilled in the art that any tools necessary for cell harvesting may be included with the device. Preferably, the set of tools are sterile. As an example only, the set of tools may include a glass vessel of separation enzyme; a sterile solution for suspension of the enzyme; a sterile nutrient solution; scalpel; forceps; syringe; medicine dropper, cell filter; wound dressings and/or spray nozzles. In a highly preferred form, the set of tools are stored in a compartment formed in the first member of the apparatus, which is covered by the second member when not in use.

In a highly preferred embodiment, the device is used to harvest a suspension of cells and apply the cells to a recipient site in the following manner.

An aliquot of sterile water is mixed with a portion of lyophilised separation enzyme and placed in the heating recess. The heating means is then activated which heats the contents (i.e. the enzyme solution) of the container to a working temperature of between 30 and 37 °C, preferably between 33 and 37°C and by way of example 37°C within 2 minutes and maintains the working temperature for at least 45 minutes. Once an operational temperature has been reached, a sample of tissue taken from a donor site is placed in the enzyme solution and incubated at the working temperature. The tissue sample is incubated for between 5 to 45 minutes. Those skilled in the art would appreciate that the time taken to achieve separation of the layers of the tissue sample is dependent on the thickness and size of the tissue sample and the incubation temperature. Once enzymatic separation of the tissue layers is achieved, the tissue sample is removed to the reservoir and the tissue layers are separated using surgical instrument/s.

A carefully measured aliquot of the second solution is then withdrawn from the fluid containment well by aspiration into a syringe and then applied to the layers. The cells between the layers of tissue are scraped off and suspended by mixing with the nutrient solution. The cell suspension is then collected, preferably using a syringe and cannula.

The harvested suspension of cells is then passed through a cell filter located in the filter recess and the filtered suspension of cells is collected into the filter recess. The reservoir may optionally be rinsed with a further volume of the second solution and this resulting suspension of cells also filtered and collected in the filter recess.

The filtered suspension of cells may then optionally be aspirated into a syringe and applied to the recipient site.

### Example 1

### Preparation of recipient site

To optimise the success of the skin graft, the wound was cleaned and assessed to be of the appropriate depth. Further, blood haemostasis was established and the wound checked for evidence of surrounding cellulitis or infection. Techniques for preparing the area included sharp dissection, dermabrasion or laser-resurfacing.

### Donor site biopsy

The donor site was chosen to appropriately match the recipient site. The donor site was infiltrated with local anaesthetic and adrenaline underneath the skin near the subcutaneous tissue. This allowed the donor site to be firm and aided in the taking a thin split-thickness biopsy. The dimensions of the biopsy were determined by the size of the surface area of the recipient site to be covered. Typically, the biopsy size has an expansion ratio of 1:10 - 1:80. In this case, a biopsy size of 2cm x 2cm was taken from the donor site giving an expansion ratio of 1:60.

### Cell Resurfacing using the Rapid Technique

Treatment of the wound was carried out using the Re-Cell^{®} Rapid Technique cell harvesting apparatus, which is explained in more detail in Example 2 below. The apparatus contained all the instruments, solutions, enzymes and dressings required for wound treatment.

The heating element was activated by depressing the "start button". Solution (sterile water for injection) (10ml) was transferred from the supplied plastic vessel marked Solution A into a glass vessel containing the separation enzyme (lyophilised trypsin) to give a final concentration of 0.5% trypsin. The enzyme solution was then mixed together, transferred to a vessel already located in the heating element recess and heated to 37°C.

The vessel containing Solution B (nutrient media) was transferred from its supplied vessel into the fluid containment well.

The previously obtained tissue sample was then placed in the enzyme solution and incubated at 37 °C for between 10 to 15 minutes. After this time, the tissue sample was removed from enzyme solution with a pair of forceps, rinsed by dipping into the fluid containment well containing Solution B and placed with the dermal side down and the epidermal side up in the reservoir.

Solution B was then aspirated from the well into a syringe and dripped from the syringe onto both layers of the biopsy.

The skin layers were separated using forceps. This allowed access to the zone of the dermal-epidermal junction of both surfaces. Cells were scraped from the surfaces to develop a plume of cells in the reservoir. The cells were then mixed in Solution B. The plume of cells was then drawn up into the syringe via a 19 gauge cannula.

The supplied filter (100µm cell filter) was mounted in the filter recess and the plume of cells in Solution B was passed through the filter. A further small amount of Solution B was then used to rinse the reservoir (eg a petri dish) and collect any remaining cells, which were also passed through the filter.

The resulting suspension of cells collected in the conical recess was aspirated into a syringe, and a nozzle was attached to the syringe for spraying or dripping on to the wound area.

The wound was re-checked to ensure that it was clean and free of debris and that there was no evidence of bacterial contamination. Further, the wound was checked to determine if haemostasis had been achieved. Once the recipient site was ready, the suspension of cells was applied to the wound surface using the nozzle.

The wound was dressed with Surfasoft™, a woven nylon dressing, which was supplied with the apparatus. The healing of the wound was followed up using standard protocols for skin-graft treatment.

### Example 2

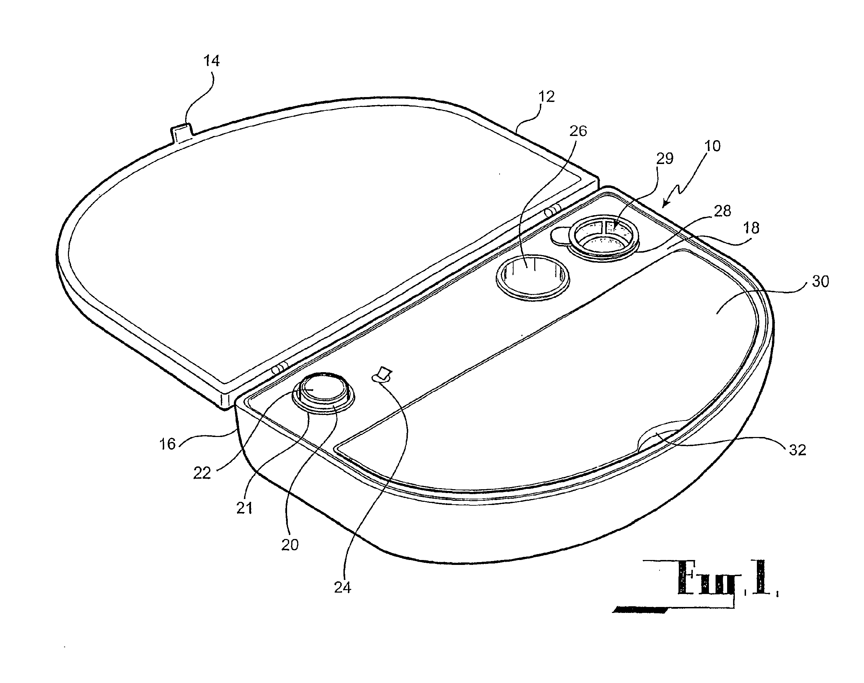

The embodiment shown in Figure 1 is directed to a Re-Cell^{®} Rapid Technique cell harvesting apparatus 10 for use in producing a transplantable cellular suspension of living tissue suitable for grafting to a patient.

As illustrated in Figure 1 the apparatus includes a closure lid 12 possessing a locking mechanism 14 adapted to releasably engage a base portion 16. The locking mechanism 14 provides a means for closing the apparatus 16 when not in use. Located within the base portion 16 is a first member 18 within which there is provided an aperture 20 in which there is located a vial 22 for the enzyme. Adjacent the aperture there is provided an activation switch 24 capable of activating the heating means (not shown). The first member also provides a fluid containment well 26 and a filter recess 28. As presented in this illustration, the filter 29 is shown to be located in the filter recess. Ordinarily, the filter is an optional item included as a separate item in the apparatus.

The aperture 20 in the first member 18 is desirably of such a diameter that it allows the neck of the vial 22 to protrude through and above the first member 18. The periphery of the aperture 20 is fitted with a collar 21 which is slightly smaller than the diameter of the body of the vial 22. Thus, when in use, the vial 22 cannot be removed from the apparatus 10 as it is held in place by the collar 21 located around the periphery of the aperture 20.

Located adjacent to the aperture 20, fluid containment well 26 and filter recess 28 is the second member 30 which is positioned on a seat (not shown) located within a storage compartment (not shown) within the first member 18. When inverted, the second member 30 forms a reservoir within which tissue manipulations may be performed. To facilitate separation of the second member 30 from the first member 18, an indent 32 is provided in the side of a portion of the second member 30, which is of such a size that a person can lift the second member from the seat on which it resides in the first member 18.

Within the filter recess 28 there is located a filter 29 (provided separately with the other components) having a mesh therein capable of separating cellular material of greater than 100µm from a cell supernatant.

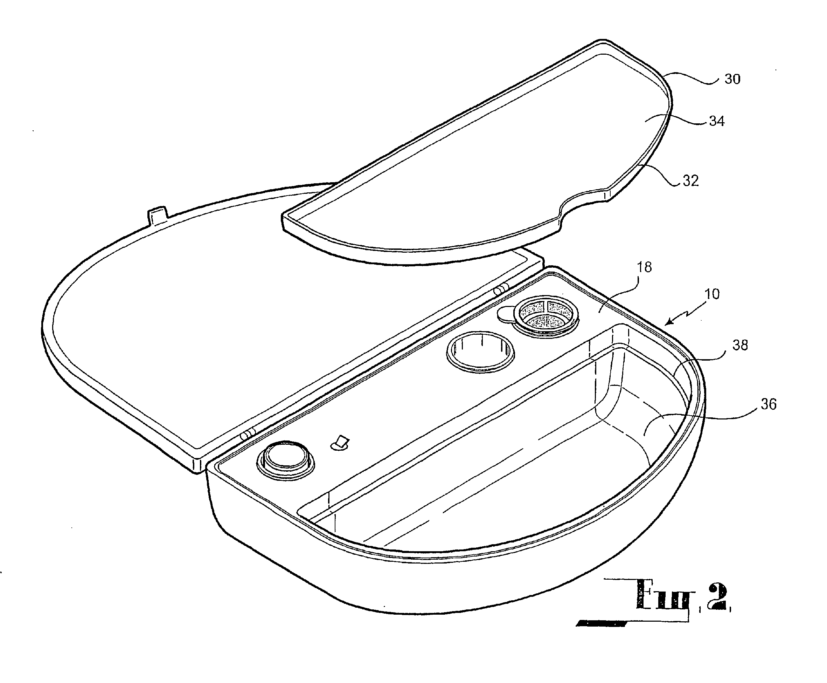

Figure 2 provides a partially exploded perspective view of the apparatus 10, wherein the second member 30 is removed from the first member 18 and inverted. Inversion of the second member 30 reveals the sidewalls 32 of the second member 30, which form the fluid containment barrier of the reservoir and a reservoir area 34 in which tissue manipulations may be performed.

Removal of the second member 30 from the first member 18 also reveals a storage compartment 36 in the first member 18, in which solutions and tools may be stored when the apparatus 10 is not in use. Within the storage compartment 36 there is located a seat 38 upon which the second member 30 may reside. The seat 38 is preferably located around the periphery of the storage compartment 36 at a depth beneath the surface of the first member 18 that is equivalent to the height of the sidewalls 32 of the second member 30.

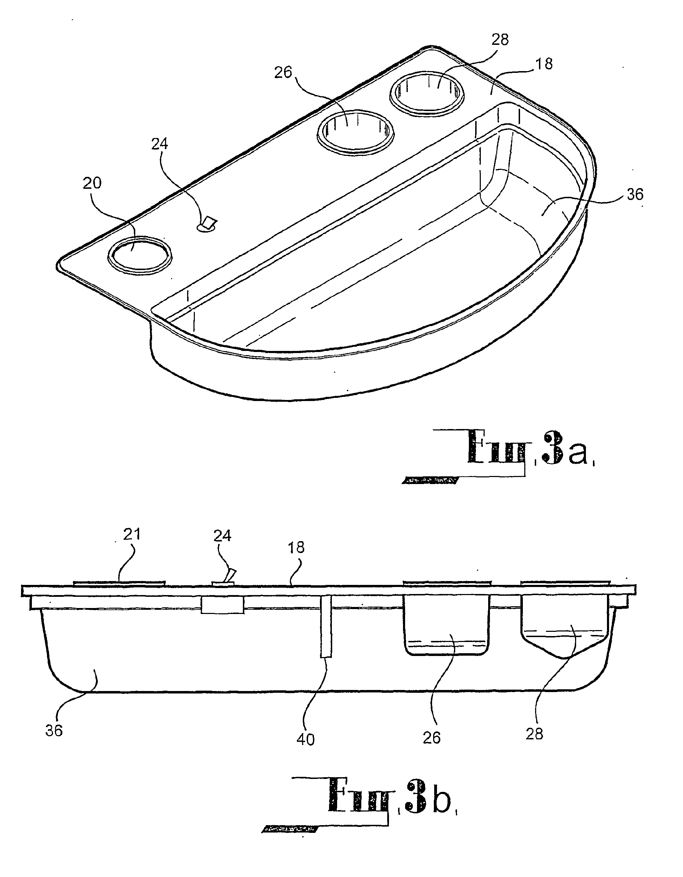

Figure 3a provides a perspective view of the first member 18 showing the storage compartment 36, the heater activating switch 24, the aperture 20, the fluid containment well 26 and filter recess 28 formed within the first member. Figure 3b provides a rear view of the first member 18 showing the filter recess 28, the fluid containment well 26, the heater activating switch 24, the aperture collar 21 and the rear wall of the storage compartment 36. As seen in this figure, the filter recess has a conical base, thereby providing a means for easy access to the cell suspension that is filtered into it. Located adjacent to the fluid containment well and on the opposite side of the filter containment well there is also provided a battery positioning member 40 which protrudes towards the base 16 of the apparatus (not shown) and provides a means for holding the batteries in place, which are required for activating the heating means.

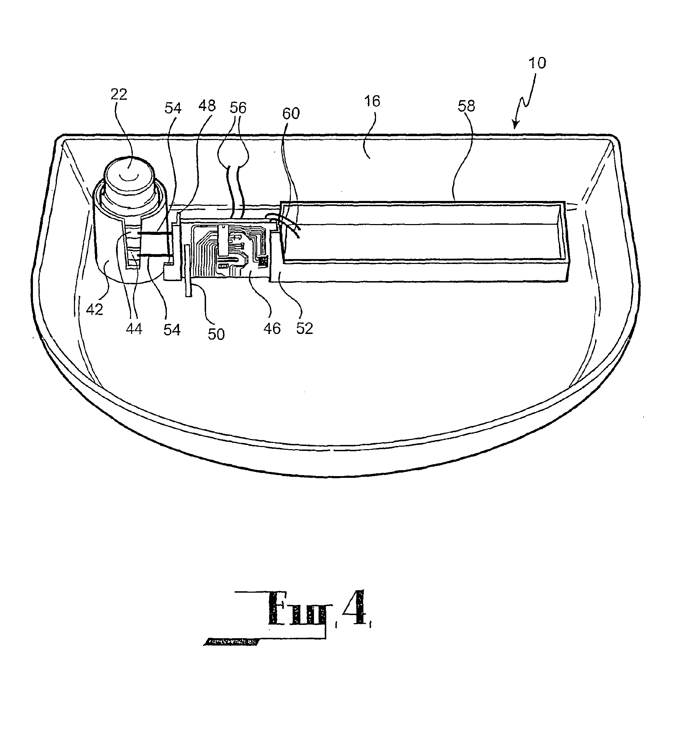

Figure 4 provides a perspective view of the base 16 of the apparatus 10 showing the vial 22 located within a containment field 42. Between the containment field 42 and the vial 22 there is located a heating collar(s) 44, which surrounds the body of the vial. Adjacent the containment field there is a circuit board 46, which is held in position by circuit board containment means 48, 50 and 52. Said circuit board 46 is in electrical communications with the heating collar(s) 44 by wires 54. The circuit board is also in electrical communication via wires 58 with the heater activating switch (not shown). Adjacent the circuit board 46 there is provided a battery containment means 58, which holds 4 AA batteries in immovable position (not shown). The batteries are in electrical communication with the circuit board 46 by wires 60. When the first member 18 is fitted to the base 16 the batteries are held in place by the battery containment means 58, the battery positioning means 40 and the base of each of the fluid containment well 26 and the filter recess 28. Preferably, the conical base of the filter recess 28 also protrudes between the batteries therein providing a further means for securing the batteries in immovable position.

## Claims

1. A method for preparing a cell suspension suitable for application to a patient, which method comprises the steps of:
(a) subjecting a skin tissue sample including cells suitable for grafting to a patient, to at least a physical and or chemical dissociating means capable of dissociating cellular stratum in the skin tissue sample for sufficient time to weaken the cohesive bonding between the tissue stratum, wherein the skin tissue sample comprises dermis, epidermis, and dermal-epidermal junction therebetween;
(b) removing the skin tissue sample from the dissociating means used in step (a) and harvesting in the presence of a nutrient solution cells from the skin tissue sample, cells suitable for grafting on to a patient wherein the nutrient solution is (i) free of xenogenic serum, (ii) capable of maintaining the viability of the cells until applied to a patient and (iii) is suitable for direct application to a region on a patient undergoing skin tissue grafting; and
(c) filtering the cell suspension produced according to step (b) to remove large cellular conglomerates;
wherein the method does not comprise the steps of pelleting and resuspending the cells.

2. A method according to claim 1, wherein the chemical dissociating means is an enzyme capable of disrupting cellular bonding.

3. A method according to claim 2, wherein the enzyme is trypsin, trypsin-EDTA, trypsin-like enzyme, dispase, collagenase, thermolysin, pronase, hyaluronidase, pancreatin, elastase and/or papain.

4. A method according to claim 1, wherein the time to weaken the cohesive bonding between the tissue stratum of step (a) is about 5 minutes to about 60 minutes.

5. A method according to claim 1, wherein the time to weaken the cohesive bonding between the tissue stratum of step (a) is about 15 minutes to about 20 minutes.

6. A method according to claim 1, wherein the nutrient solution is Hartmann's solution.

7. Cell suspension obtainable by a method according to claim 1 for use in the treatment of a patient in need of skin graft surgery, wherein the cell suspension comprises cells capable of reproduction, including keratinocyte basal cells, melanocytes and fibroblasts, wherein the cells are in a ratio to each other comparable to those seen in situ.

8. The cell suspension for the use according to claim 7, wherein the cell suspension comprises cells autologous to the patient.

9. A cellular suspension for use in the treatment of a patient in need of skin graft surgery, which cellular suspension is prepared according to the following steps:
(a) subjecting a skin tissue sample including cells suitable for grafting to a patient, to an enzyme suitable for dissociating cohesive pieces of the tissue stratum in the skin tissue sample, wherein the skin tissue sample comprises dermis, epidermis, and dermal-epidermal junction therebetween;
(b)removing the skin tissue sample from the enzyme solution used in step (a) and harvesting in the presence of a nutrient solution cells from the skin tissue sample, which cells are suitable for grafting on to a patient wherein the nutrient solution is (i) free of xenogenic serum, (ii) capable of maintaining the viability of the cells until applied to a patient and (iii) is suitable for direct application to a region on a patient undergoing skin tissue grafting;
(c) filtering the cell suspension produced according to step (b) to remove large cellular conglomerates;
wherein the cell suspension comprises cells capable of reproduction, including keratinocyte basal cells, melanocytes and fibroblasts, wherein the cells are in a ratio to each other comparable to those seen in situ.

10. The cellular suspension for the use according to claim 9 wherein the nutrient solution is Hartmann's solution.

## Patentansprüche

1. Ein Verfahren zum Präparieren einer Zellsuspension geeignet für die Verabreichung an einen Patienten, wobei das Verfahren die Schritte enthält von:
(a) Aussetzen einer Hautgewebeprobe, die Zellen beinhaltet, die für eine Transplantation in einen Patienten geeignet sind, mindestens einem physikalischen und oder chemischen Dissoziierungsmittel, das fähig ist, zelluläres Stratum in der Hautgewebeprobe zu dissoziieren über eine ausreichende Zeit, um die kohäsive Verbindung zwischen dem Gewebestratum zu schwächen, wobei die Hautgewebeprobe Dermis, Epidermis und dermale-epidermale Verbindung dazwischen enthält;
(b) Entfernen der Hautgewebeprobe von dem Dissoziierungsmittel verwendet in Schritt (a) und Ernten von Zellen in Gegenwart einer Nährlösung aus der Hautgewebeprobe, wobei die Zellen geeignet sind für eine Transplantation in einen Patienten, wobei die Nährlösung (i) frei ist von xenogenem Serum, (ii) fähig ist, die Lebensfähigkeit der Zellen aufrecht zu erhalten bis sie an einem Patienten verabreicht werden und (iii) geeignet ist für die unmittelbare Anwendung in einem Bereich an einem Patient, der sich einer Hautgewebetransplantation unterzieht; und
(c) Filtrieren der Zellsuspension erzeugt gemäß Schritt (b), um große zelluläre Konglomerate zu entfernen;
wobei das Verfahren keine Schritte des Pelletierens und Resuspendierens der Zellen enthält.

2. Ein Verfahren gemäß Anspruch 1, wobei das chemische Dissoziierungsmittel ein Enzym ist, das fähig ist, die zelluläre Verbindung zu zerreißen.

3. Ein Verfahren gemäß Anspruch 2, wobei das Enzym Trypsin, Trypsin-EDTA, Trypsin-ähnliches Enzym, Dispase, Kollagenase, Thermolysin, Pronase, Hyaluronidase, Pancreatin, Elastase und/oder Papain ist.

4. Ein Verfahren gemäß Anspruch 1, wobei die Zeit, um die kohäsive Verbindung zwischen dem Gewebestratum in Schritt (a) zu schwächen, ungefähr 5 Minuten bis ungefähr 60 Minuten beträgt.

5. Ein Verfahren gemäß Anspruch 1, wobei die Zeit, um die kohäsive Verbindung zwischen dem Gewebestratum in Schritt (a) zu schwächen, ungefähr 15 Minuten bis ungefähr 20 Minuten beträgt.

6. Ein Verfahren gemäß Anspruch 1, wobei die Nährlösung eine Hartmann-Lösung ist.

7. Zellsuspension erhältlich durch ein Verfahren gemäß Anspruch 1 zur Verwendung in der Behandlung eines Patienten, der eine Hauttransplantationschirugie benötigt, wobei die Zellsuspension Zellen enthält, die fähig sind, sich zu reproduzieren, einschließlich Keratinozyten-Basalzellen, Melanozyten und Fibroblasten, wobei die Zellen in einem Verhältnis zueinander sind vergleichbar mit demjenigen wie es in situ zu sehen ist.

8. Die Zellsuspension zur Verwendung gemäß Anspruch 7, wobei die Zellsuspension für den Patienten autologe Zellen enthält.

9. Eine zelluläre Suspension zur Verwendung in der Behandlung eines Patienten, der eine Hauttransplantationschirugie benötigt, wobei die zelluläre Suspension gemäß den folgenden Schritten präpariert wird:
(a) Aussetzen einer Hautgewebeprobe, die Zellen beinhaltet, die für eine Transplantation in einen Patienten geeignet sind, einem Enzym, das geeignet ist, in einer Hautgewebeprobe kohäsive Stücke aus dem Gewebestratum zu dissoziieren, wobei die Hautgewebeprobe Dermis, Epidermis und dermale-epidermale Verbindungen dazwischen enthält;
(b) Entfernen der Hautgewebeprobe von der Enzymlösung verwendet in Schritt (a) und Ernten von Zellen in Gegenwart einer Nährlösung aus der Hautgewebeprobe, wobei die Zellen geeignet sind für eine Transplantation in einen Patienten, wobei die Nährlösung (i) frei ist von xenogenem Serum, (ii) fähig ist, die Lebensfähigkeit der Zellen aufrecht zu erhalten bis sie an einem Patienten angewendet werden und (iii) geeignet ist für die unmittelbare Anwendung in einem Bereich an einem Patient, der sich einer Hautgewebetransplantation unterzieht;
(c) Filtrieren der Zellsuspension erzeugt gemäß Schritt (b), um große zelluläre Konglomerate zu entfernen;
wobei die Zellsuspension Zellen enthält, die fähig sind, sich zu reproduzieren, einschließlich Keratinozyten-Basalzellen, Melanozyten und Fibroblasten, wobei die Zellen in einem Verhältnis zueinander sind vergleichbar mit demjenigen wie es in situ zu sehen ist.

10. Die zelluläre Suspension für die Verwendung gemäß Anspruch 9, wobei die Nährlösung eine Hartmann-Lösung ist.

## Revendications

1. Procédé de préparation d'une suspension cellulaire appropriée pour une application à un patient, lequel procédé comprend les étapes consistant à :
(a) soumettre un échantillon de tissu cutané comprenant des cellules appropriées pour une greffe à un patient, à au moins un moyen de dissociation physique et/ou chimique capable de dissocier la couche cellulaire dans l'échantillon de tissu cutané pendant un temps suffisant pour affaiblir les liaisons cohésives entre la couche tissulaire, où l'échantillon de tissu cutané comprend le derme, l'épiderme et la jonction dermo-épidermique entre les deux ;
(b) retirer l'échantillon de tissu cutané du moyen de dissociation utilisé dans l'étape (a) et récolter en présence d'une solution nutritive les cellules provenant de l'échantillon de tissu cutané, les cellules appropriées pour une greffe à un patient où la solution nutritive est (i) dépourvue de sérum xénogénique, (ii) capable de maintenir la viabilité des cellules jusqu'à leur application à un patient et (iii) appropriée pour une application directe sur une région d'un patient subissant une greffe de tissu cutané ; et
(c) filtrer la suspension cellulaire produite selon l'étape (b) pour éliminer les gros conglomérats cellulaires ;
où le procédé ne comprend pas les étapes de centrifugation et de remise en suspension des cellules.

2. Procédé selon la revendication 1, dans lequel le moyen de dissociation chimique est une enzyme capable de rompre les liaisons cellulaires.

3. Procédé selon la revendication 2, dans lequel l'enzyme est la trypsine, la trypsine-EDTA, une enzyme de type trypsine, la dispase, la collagénase, la thermolysine, la pronase, la hyaluronidase, la pancréatine, l'élastase et/ou la papaïne.

4. Procédé selon la revendication 1, dans lequel le temps pour affaiblir les liaisons cohésives entre la couche tissulaire de l'étape (a) est d'environ 5 minutes à environ 60 minutes.

5. Procédé selon la revendication 1, dans lequel le temps pour affaiblir les liaisons cohésives entre la couche tissulaire de l'étape (a) est d'environ 15 minutes à environ 20 minutes.

6. Procédé selon la revendication 1, dans lequel la solution nutritive est la solution de Hartmann.

7. Suspension cellulaire pouvant être obtenue par un procédé selon la revendication 1 pour une utilisation dans le traitement d'un patient ayant besoin d'une chirurgie de greffe cutanée, où la suspension cellulaire comprend des cellules capables de reproduction, y compris des cellules basales de kératinocytes, des mélanocytes et des fibroblastes, où les cellules sont dans un rapport les unes par rapport aux autres comparable à celui observé *in situ.*

8. Suspension cellulaire pour une utilisation selon la revendication 7, où la suspension cellulaire comprend des cellules autologues par rapport au patient.

9. Suspension cellulaire pour une utilisation dans le traitement d'un patient ayant besoin d'une chirurgie de greffe cutanée, laquelle suspension cellulaire est préparée selon les étapes suivantes :
(a) la soumission d'un échantillon de tissu cutané comprenant des cellules appropriées pour une greffe à un patient, à une enzyme appropriée pour dissocier les parties cohésives de la couche tissulaire dans l'échantillon de tissu cutané, où l'échantillon de tissu cutané comprend le derme, l'épiderme et la jonction dermo-épidermique entre les deux ;
(b) le prélèvement de l'échantillon de tissu cutané à partir de la solution enzymatique utilisée dans l'étape (a) et la récolte en présence d'une solution nutritive des cellules provenant de l'échantillon de tissu cutané, lesquelles cellules sont appropriées pour une greffe à un patient où la solution nutritive est (i) dépourvue de sérum xénogénique, (ii) capable de maintenir la viabilité des cellules jusqu'à leur application à un patient et (iii) appropriée pour une application directe sur une région d'un patient subissant une greffe de tissu cutané ;
(c) la filtration de la suspension cellulaire produite selon l'étape (b) pour éliminer les gros conglomérats cellulaires ;
où la suspension cellulaire comprend des cellules capables de reproduction, y compris des cellules basales de kératinocytes, des mélanocytes et des fibroblastes, où les cellules sont dans un rapport les unes par rapport aux autres comparable à celui observé *in situ.*

10. Suspension cellulaire pour une utilisation selon la revendication 9, dans laquelle la solution nutritive est la solution de Hartmann.
